# EUROPÄISCHE PATENTSCHRIFT

(11) **EP 1 802 277 B1**
(45) Veröffentlichungstag und Bekanntmachung des Hinweises auf die Patenterteilung: **13.01.2010**
(21) Anmeldenummer: 05802312.8
(22) Anmeldetag: 14.10.2005
(51) Int. Cl.: A61K 9/127

(54) **LIPOSOMALE ZUSAMMENSETZUNG EINEN WIRKSTOFF ZUR RELAXIERUNG GLATTER MUSKULATUR ENTHALTEND, DIE HERSTELLUNG DIESER ZUSAMMENSETZUNG UND DEREN THERAPEUTISCHE VERWENDUNG**
LIPOSOMAL COMPOSITION COMPRISING AN ACTIVE INGREDIENT FOR RELAXING SMOOTH MUSCLEs AND THERAPEUTIC USE OF SAID COMPOSITION
COMPOSITION LIPOSOMALE CONTENANT UN PRINCIPE ACTIF DESTINE A RELAXER LA MUSCuLATURE LISSE ET L'EMPLOI THÉRAPEUTIQUE DE CETTE COMPOSITION

(30) Priorität: 18.10.2004 EP 04024753
(43) Veröffentlichungstag der Anmeldung: 04.07.2007
(73) Patentinhaber: Polymun Scientific Immunbiologische Forschung GmbH, 1190 Wien (AT)
(72) Erfinder: WAGNER, Andreas, A-2500 Baden (AT); VORAUER-UHL, Karola, A-1220 Wien (AT); KATINGER, Hermann, A-1190 Wien (AT)
(74) Vertreter: Bogensberger, Burkhard
(86) Internationale Anmeldenummer: PCT/EP2005/011054
(87) Internationale Veröffentlichungsnummer: WO 2006/042701

(56) Entgegenhaltungen:
- WO-A-03/059320
- US-A1- 2004 014 761

## Beschreibung

### TECHNISCHES GEBIET

Die vorliegende Erfindung bezieht sich auf pharmazeutische Zusammensetzungen auf der Basis von topisch applizierbaren Wirkstoffen in Liposomen, die die Durchblutung von Geweben, insbesondere im Genitalbereich, verstärken, sowie auf die Herstellung solcher Zusammensetzungen und deren Anwendung.

### HINTERGRUND DER ERFINDUNG

Liposomen sind als Mittel zur kontrollierten Freisetzung pharmazeutischer Wirkstoffe bekannt (siehe z.B. Übersicht bei Ulrich, Biosci Rep. 2002; 22(2): 129-50), oder WO 96/14083 für SOD in Liposomen. Auch die Formulierung von Lokalanästhetika in topisch applizierten Liposomen ist dem Fachmann bekannt; z. B. beschreibt US-4937078 Liposomen, die übliche Natriumkanal-Blocker wie Tetracain, Lidocain usw. enthalten. Weiters sind chemische Verbindungen bekannt, die die Gewebedurchblutung fördern und und vor allem durch ihren Einsatz zur Behebung erektiler Störungen und Impotenz bekannt geworden sind (siehe z.B. WO 94/28902 und EP 0967214 A1).

So beschreibt beispielsweise US 20040014761_A1 die Behandlung weiblicher Sexualfunktionsstörungen durch topische Applikation einer Zusammensetzung auf Basis eines Trägermaterials und eines Phophodiesterasehemmers als Wirkstoff. Konkret wird für den Wirkstoff Tadalafil als ein mögliches Trägermaterial eine liposomale Mischung aus verschiedenen Pflanzenölen, Ei-Phospholipiden und Glyzerin offenbart.

In WO03/059320 A1 wiederum wird eine liposomale Zusammensetzung auf Basis von Lidocain und einem Lokalanästhetikum offenbart, die zur Behandlung anorektaler Störungen verwendet werden kann. Diese Zusammensetzung kann gegebenenfalls auch Sildenafil enthalten.

### KURZE BESCHREIBUNG DER ERFINDUNG

Während die bekannten Wirkstoffpräparate auf der Basis von Pyrazolopyrimidonen und Pyrazolopyrimidinonen entweder als peroral oder intranasal applizierbare Formulierungen vorliegen, ist es das Ziel der vorliegenden Erfindung, eine Zusammensetzung zur wirksamen topischen Anwendung solcher Substanzen, vorzugsweise direkt im Genitalbereich, bereit zu stellen, insbesondere eine Formulierung, welche eine insgesamt niedrige, gleichzeitig jedoch ausreichend hohe lokale Dosierung dieser Wirkstoffe im Bereich der weiblichen oder männlichen Geschlechtsorgane erlaubt.

Erreicht wird dieses Ziel erfindungsgemäss durch die Bereitstellung eines liposomalen Systems zur topischen, insbesondere transdermalen und/oder transmucosalen, Verabreichung von Wirkstoffen, die eine Relaxation der glatten Muskulatur, speziell jener der zuführenden Blutgefässe in den Geschlechtsorganen, bewirken. Eine solche Wirkung kann z.B. durch eine induzierte Ausschüttung von Calcium-lonen ausgelöst werden.

### DETAILLIERTE BESCHREIBUNG DER ERFINDUNG

In einer ersten Ausführungsform bezieht sich die Erfindung auf eine Formulierung, worin der Wirkstoff, vorzugsweise aus der Gruppe der Prostaglandine, Adenylatcyolasen, cAMP, AMP, ATP, NO-Synthetasen, Stickstoffmonoxid (NO), NO-Verbindungen, Nitrate, Guanylatcyclasen, cGMP, GMP, GTP und Phosphodiesterasen, insbesondere Sildenafil, Tadalafil und Vardenafil, in Liposomen eingeschlossen und/oder an Liposomen gebunden vorliegt.

Durch die erfindungsgemässe liposomale Formulierung wird nicht nur ein temporäres Wirkstoff-Depot im umliegenden Gewebe, aus dem kontinuierlich Substanz abgegeben wird, sondern ausserdem eine bessere Bioverfügbarkeit und längere Halbwertszeit im Vergleich zur systemischen Anwendung erzielt.

Durch die relaxierende Wirkung auf die glatten Muskelzellen kommt es zu einer verstärkten Durchblutung der äusserlich behandelten Gewebe, beispielsweise der Geschlechtsorgane, und in der Folge zu einer gesteigerten Sensitivität und Empfindsamkeit bei sexuellen Aktivitäten.

Wirkstoffe oder wirksame Substanzen im Sinne der vorliegenden Erfindung sind vor allem jene Substanzen, die in den cAMP- oder cGMP-Kreislauf eingreifen und eine erhöhte Ca-Ionen-Ausschüttung hervorrufen. Dazu gehören beispielsweise Substanzen wie Papaverin, Phentolamin, die den cAMP-Weg stimulieren; Stickstoffmonoxid (NO), welches eine wichtige Transmitterfunktion ausübt und eine Guanylatcyclase aktiviert, die ihrerseits wiederum cGMP bildet; NO-Donatoren; Nitroglycerin; Minoxidil; L-Arginin; Linsidomin (wird im Körper durch NO-Synthetase durch Umwandlung von Arginin zu Citrullin hergestellt); Molsidomin; Phosphodiesteraseinhibitoren wie z.B. Sildenafil oder Sildenafilcitrat, welches in den cGMP-Weg eingreift, wobei ein PDE5-Rezeptor daran beteiligt ist; Prostaglandine wie z.B. Alprostadil (PGE-1), Dinoprostone (PGE-2), die in den cAMP-Kreislauf eingreifen.

Soweit im Nachfolgenden Sildenafil erwähnt wird, sind damit auch Tadalafil, Vardenafil sowie deren saure Salze, z.B. Sildenafilcitrat und Vardenafil-HCI 3H2O (Vardenafil-Hydrochloridtrihydrat), gemeint, soweit sich aus dem jeweiligen Sinnzusammenhang nicht etwas anderes ergibt.

Zur Herstellung und Beladung der Liposomen mit Wirkstoff hat sich das in der WO 02/36257 offenbarte, skalierbare Verfahren zur Wirkstoff-Verkapselung aufgrund seiner hohen Effizienz bei gleichzeitig äusserst schonenden Verfahrensbedingungen als besonders vorteilhaft erwiesen. Mit diesem Verfahren werden typischerweise unilamellare Liposomen mit einer Lipiddoppelschichtmembran erzeugt, deren ausgesprochen gute Hautpenetrationsfähigkeit bereits in früheren Arbeiten erkannt und nachgewiesen wurde. Andere im Stand der Technik bekannte Verfahren zur Herstellung und Beladung von Liposomen können aber ebenfalls angewandt werden.

Eine maximale Beladungsdichte lässt sich durch aktive Beladung der Liposomen mit Wirkstoffen erzielen. Dieser Vorgang kann in zwei Hauptkategorien unterteilt werden: Beladung der Membran und Beladung der intraliposomalen wässrigen Phase. Wirkstoffe, die protonierbare Gruppen, beispielsweise Aminogruppen, enthalten, können durch H⁺-Gradienten-gesteuerte Beladung in Liposomen eingeschlossen und dann im protonierten Zustand dort zurück gehalten werden. Für diese Art der aktiven Beladung ist das wichtigste Merkmal der Liposomenmembran/Liposomenmedium-Verteilungskoeffizient. Es wurde gefunden, dass der Octanol/Buffer-Verteilungskoeffizient einen guten Hinweise für die Transmembran-Diffusion eines Stoffes liefert und daher für die Beladung mit Wirkstoff beziehungsweise für das Freisetzungsprofil relevant ist.

Unter Zugrundelegung dieses theoretischen Modells wurden Liposomen mit unterschiedlicher Lipidzusammensetzung, vorzugsweise mit langkettigen Phospholipiden und geringen Cholesterin-Konzentrationen), in einem geeigneten Beladungspuffer, vorzugsweise in einem Ammoniumsulfat- oder Zitronensäure/Natriumcarbonat-Puffer, hergestellt. Nach der Herstellung der Liposomen in Ammoniumsulfat- oder Citratpuffer wird das umgebende Medium modifiziert, d.h. ausgetauscht oder verdünnt, gegebenenfalls neutralisiert oder alkalisiert, und dadurch zwischen dem intraliposomalen Puffer und dem extraliposomalen Medium ein H⁺-Gradient erzeugt. Nach Zusatz eines Wirkstoffes in das extraliposomale Medium migriert der Wirkstoff dank dieses H⁺-Gradienten in die Liposomen hinein, wird dort protoniert und verbleibt stabil in den Liposomen.

Bei Anwendung dieser Technik wird das Ausmass der Beladung bzw. die Beladungskapazität in erster Linie vom Verhältnis der H⁺-Konzentrationen innerhalb und ausserhalb der Liposomen bestimmt. In den durchgeführten Versuchen konnten mit Wirkstoff/Lipid-Verhältnissen im Bereich von 200 - 400 nmol Wirkstoff je µmol Lipid ähnliche Werte erzielt werden, wie sie aus der Literatur über aktiv beladene Liposomen bekannt sind. Eine Erhöhung der Wirkstoffkonzentration im Beladungsmedium führte zu keiner Steigerung der Beladungskapazität.

Die zuvor beschriebene aktive Beladung ist ein dreistufiger Vorgang, bestehend aus Vesikelbildung, Wirkstoffzugabe und Alkalisierung. Es war daher ein weiteres Ziel der Erfindung, ein einstufiges Herstellungsverfahren zu etablieren, welches unter Verwendung des in der WO 02/36257 offenbarten Kreuzstrommoduls realisiert werden konnte. Zu diesem Zweck wurde der Wirkstoff in einer H⁺-reichen, wässrigen Phase, z.B. Ammoniumsulfatlösung oder Zitronensäurelösung gelöst, mittels Kreuzstrom-Injektionstechnik in Liposomen eingeschlossen und unmittelbar anschliessend darauf die verbleibende externe (= extraliposomale), wässrige Phase mit einem Verdünnungspuffer (z.B. 5% Glucoselösung beim Ammoniumsulfatsystem bzw. Zitronensäure/Natriumcarbonat pH 9,0 - 9,5 beim Citratsystem) verdünnt. Es wurde gefunden, dass die Qualität der wirkstoffbeladenen Liposomen allein schon durch Variation, insbesondere durch Verringerung, des Cholesteringehaltes in der Vesikelmembran verbessert werden kann, vor allem in Bezug auf deren Hautpenetrationsfähgkeit.

Wo erforderlich oder gewünscht, kann durch Anhebung der mittleren Liposomengrösse von ca. 150 - 200 nm (wie in den hierin beschriebenen Experimenten zumeist verwendet) auf 300 bis 500 nm, die Beladungskapazität weiter erhöht werden. Zusätzlich lässt sich auch die Effizienz des Verfahrens, d.h. die Menge an liposomal eingeschlossenem Wirkstoff je ml Suspension, durch Erhöhung der Lipidkonzentration entweder während der Herstellung oder bei der anschliessenden Filtration der Vesikel noch weiter steigern.

Wenn Sildenafil als Wirkstoff eingesetzt wird, ist zur aktiven Beladung das Ammoniumsulfat/Glucoselösung-System vorzuziehen, da Sildenafil in Citratpuffer nur schlecht oder gar nicht löslich ist. NH3, das mit Ammoniumsulfat im intraliposomalen wässrigen Milieu in einem reversiblen Gleichgewicht vorliegt, hat das Bestreben, durch die Liposomenmembran hinauszuwandern und dabei ein H⁺ zurückzulassen. Sildenafil wandert im Gegenzug in das Liposom hinein, nimmt dabei das Wasserstoffion H⁺ auf, wird dadurch hydrophiler und verbleibt damit innerhalb der Membran. Auf diese Weise kann Sildenafil effizient in Liposomen geladen werden. Dies gilt in ähnlicher Weise auch für die Sildenafil-Alternativen Tadalafil und Vardenafil.

Zur Ermittlung der besten Liposomenformulierung in Bezug auf Membranflexibilität und die damit verbundenen Hautpenetrationseigenschaften wurden verschiedene Liposomensuspensionen mit unterschiedlicher Lipidzusammensetzung vorbereitet und getestet. Dabei wurden in erster Linie Phospholipide, gegebenenfalls in Kombination mit Cholesterin, eingesetzt. Es liegt jedoch im Rahmen der Erfindung, Phospholipide durch andere Lipide zu ersetzen oder zu ergänzen, beispielsweise durch Glykolipide, Cerebroside, Sulfatide oder Galactoside. Typische Vertreter der einsetzbaren Lipide sind z.B. Phosphatidylethanotamin, Phosphatidylcholin, Phosphatidylserin, Phosphatidylinositol, Phosphatidylglycerol, Cardiolipin, Sphingomyeline, Plasmalogene, Glyceroglykolipide, Ceramid, Glykosphingolipide, neutrale Glykosphingolipide.

Eine Möglichkeit, die für transdermale Applikationen bedeutsame Membranfluidität zu verbessern, besteht darin, den Phasenübergang der liposomalen Doppelschichtmembran zu reduzieren, welcher vornehmlich durch die Länge der Acylketten der Phospholipide, die Menge an Cholesterin und die Sättigung der Phospholipide bestimmt wird. Aus diesem Grunde wurde in einer Ausführungsform des Herstellungsverfahrens DPPC, ein Phospholipid mit einer Acylkettenlänge von 16 Kohlenstoffatomen, durch DMPC (Kettenlänge von 14 Kohlenstoffatomen) ersetzt, welches die Schmelztemperatur TM von ca. 45°C auf 31°C erniedrigt.

Eine zweite Möglichkeit, die Membranrigidität zu senken und die Fluidität zu erhöhen besteht darin, den Cholesterin-Anteil in der Membran zu vermindern. Ausgehend von einem Verhältnis DPPC : Cholesterin von 55 : 45 Mol% (wie in der Literatur für Liposomenbeladung beschrieben), wurde die Cholesterinmenge sukzessive auf 38 bzw. 30 %, bezogen auf den Gesamtlipidgehalt, reduziert. Es konnte eine leichte Abnahme der Wirkstoffbeladung festgestellt werden, im Vergleich zu Ergebnissen mit höheren Cholesterin-Gehalten. Diese Liposomen zeigten jedoch verbesserte Hautpenetrationseigenschaften und blieben auch im Langzeitversuch über Wochen stabil ohne nennenswerten Wirkstoffverlust.

Entgegen Erkenntnissen aus früheren Arbeiten konnten auch cholesterinfreie Liposomen stabil hergestellt und erfolgreich mit Wirkstoff beladen werden, sodass erfindungsgemäss der Cholesteringehalt in einem Bereich von 0 - 50 Mol%, bezogen auf den Gesamtlipidgehalt, liegt.

Eine dritte Möglichkeit, liposomale Membranen flexibler zu machen, besteht darin, die voll gesättigten DPPC- oder DMPC-Lipide durch Hühnerei-Phosphatidylcholin (E-PC), eine natürliche Lipidmischung mit ungesättigten Phospholipiden, zu ersetzen. Neben Stabilitätsproblemen bei Verwendung dieser natürlichen Lipide war es ausserdem erforderlich, die Liposomen unter Stickstoffatmosphäre herzustellen. Trotzdem ergaben diese Vesikel weder gute Resultate in Bezug auf die Vesikelgrösse und -homogenität, noch Verbesserungen in den Hautpenetrationseigenschaften.

Es liegt im Rahmen der vorliegenden Erfindung, auch alternative, dem Fachmann bekannte, funktionell äquivalente Systeme zur Ausbildung eines H⁺-Gradienten einzusetzen. Unter "funktionell äquivalent" ist in diesem Zusammenhang die Fähigkeit zu verstehen, über die Lipiddoppelschichtmembran der Liposomen hinweg einen H⁺-Gradienten ausbilden zu können und dabei die Membranintegrität nicht zu zerstören, sodass eingeschlossener, insbesondere protonierter, Wirkstoff stabil - im Sinne der hierin offenbarten Stabilitätskriterien - in den Liposomen verbleibt.

Zur Anwendung einer liposomalen Sildenafilzusammensetzung als topisch einzusetzendes Therapeutikum werden die Liposomen bevorzugt in ein Hydrogel eingemischt, welches leichter auf die Haut aufzutragen ist, als eine reine Suspension. Es liegt jedoch im Rahmen der vorliegenden Erfindung, auch andere galenische Formulierungen für die Sildenafil-Liposomen herzustellen und topisch anzuwenden, insbesondere Formulierungen in Form von Lösungen, Lotionen, Emulsionen, Tinkturen, Sprays, Salben oder Cremes. Dem Fachmann auf dem Gebiet sind weitere Möglichkeiten vertraut, ebenso wie die dazu erforderlichen, pharmazeutisch zulässigen Begleit- und Zusatzstoffe zur Herstellung der verschiedenen galenischen Formulierungen.

In früheren Experimenten bewährt hat sich beispielsweise Carbopol 981 NF (Fa. Noveon), ein Hydrogel, welches in sehr geringen Konzentrationen eingesetzt werden kann. Es ist für den pharmazeutischen Einsatz zugelassen, relativ billig zu erwerben und in grossen Mengen verfügbar.

### KURZE BESCHREIBUNG DER ABBILDUNG

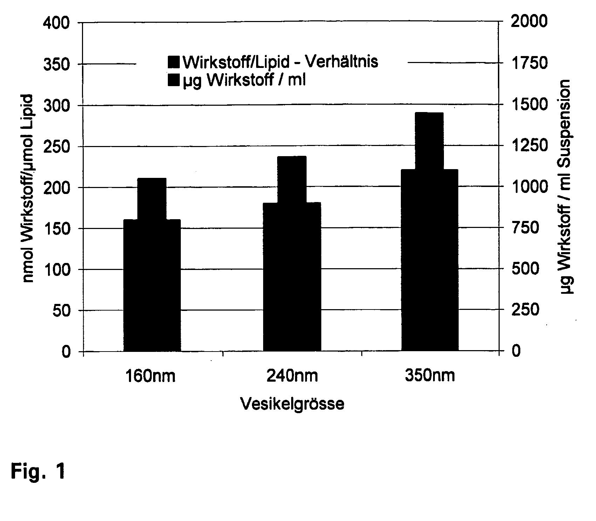

- Abb.1: zeigt eine graphische Darstellung der Einschlussmengen von Sildenafil in DPPC/Cholesterin-Liposomen in Abhängigkeit von der Vesikelgrösse.

Zur besseren Illustration wird die Erfindung anhand der nachfolgenden Beispiele weiter erläutert.

### Beispiel 1: Herstellung von Sildenafil-Liposomen

Die Liposomen werden bevorzugt nach dem bekannten Crossflow-Verfahren (WO 02/36257) unter Verwendung einer für den gewünschten Wirkstoff geeigneten wässrigen Phase hergestellt, wobei optional zumindest ein Teil des Wirkstoffes in dieser wässrigen Phase vorgelegt und im Zuge der Liposomenentstehung im Inneren der Liposomen eingeschlossen wird. Durch die nachfolgende Verdünnung der Liposomensuspension mittels neutralem oder alkalischem Verdünnungspuffer, der vorzugsweise ebenfalls Wirkstoff enthält, wird ein H⁺-Gradient zwischen der Innen- und Aussenseite der Liposomen erzeugt, der sowohl weiteren protonierbaren Wirkstoff aus dem Verdünnungspuffer rasch und effizient in die Liposomen transportiert als auch den im Zuge der Liposomenentstehung bereits eingeschlossenen Wirkstoff zurück hält.

Alternativ dazu kann auch ein Verfahren gewählt werden, bei dem in einem ersten Schritt Puffer-gefüllte Liposomen ohne Wirkstoff erzeugt werden und der Wirkstoff erst anschliessend an die Liposomenherstellung aktiv über einen H⁺-Gradienten, wie zuvor beschrieben, in die Liposomen geladen wird. Diese Vorgangsweise erlaubt es, die Qualität der Liposomensuspension vor der Beladung mit Wirkstoff zu überprüfen.

Beide Techniken sind sehr gut reproduzierbar und erlauben das Einschliessen beliebiger Wirkstoffe in Liposomen. Sie sind ausserdem unter ausgesprochen mitden Verfahrensbedingungen ausführbar und erlauben es, auf den Einsatz möglicherweise schädigender Lösungsmittel sowie insbesondere auf die Anwendung von Scherkräften zur Vesikelbildung vollständig zu verzichten.

Darüber hinaus ist es mit dieser Cross-Flow Methode möglich, sämtliche Reagenzien in steriler oder keimfreier Form bereitzustellen und die Liposomenherstellung und -beladung unter aseptischen Bedingungen durchzuführen, sodass schlussendlich ein steriles oder keimfreies Produkt in Form wirkstoffbeladener Liposomen resultiert.

Liposomenherstellung (nach WO 02/36257) im Einzelnen:

Die Lipidmischung wird in 96% Ethanol und je nach Lipidauswahl bzw. Lipidzusammensetzung bei einer Temperatur im Bereich von 25 bis 60°C, beispielsweise im Falle von DPPC-Liposomen bei einer Temperatur von 50 bis 55°C, unter Rühren gelöst. Auch die Pufferlösungen werden vorzugsweise auf dieselbe Temperatur, beispielsweise 55°C, temperiert. Während die polare, wässrige Phase (Puffer) durch das Kreuzstrommodul mittels einer Pumpe, z.B. einer Peristaltikpumpe, gepumpt wird, wird gleichzeitig die Ethanol/Lipid-Lösung unter beliebig vorwählbarem Druck in die polare Phase injiziert.

Erste Versuche haben gezeigt, dass zum Herstellen des H⁺-Gradienten, der das aktive Beladen ermöglicht, je nach eingesetztem Wirkstoff verschiedene Puffersysteme auch verschieden gut geeignet sind. So hat sich beispielsweise bei Verwendung von Sildenafilcitrat als Wirkstoff ein Puffersystem von Zitronensäure (intraliposomal) und ein äquimolarer, neutraler oder leicht basischer Puffer, wie z.B. Zitronensäure/Na-Carbonat pH 7,5-8,0 (extraliposomal) als nicht günstig erwiesen, da Sildenafilcitrat in Puffersystemen dieser Art nicht oder kaum löslich ist. Sildenafilcitrat löst sich hingegen sehr gut in Wasser, weshalb für diesen Wirkstoff das aktive Beladen mit Ammoniumsulfatgradienten bevorzugt wird.

Mit dieser Methode werden daher Liposomen bevorzugt in Gegenwart eines Ammoniumsulfatpuffers (vorzugsweise 125 mmol) gebildet. Nach der Vesikelbildung wird die ausserhalb der Liposomen verbliebene wässrige Phase, hier die Ammoniumsulfatlösung, modifiziert, beispielsweise mittels Verdünnungspuffer verdünnt oder mittels Diafiltration durch eine 5% Glucoselösung ersetzt, wodurch kleine amphiphile Moleküle wie Sildenafil in die Liposomen geladen werden können und dort protoniert werden, während im Gegenzug NH3 aus den Liposomen entweicht.

### a) zweistufige Variante:

Externe Beladung von Liposomen mit Sildenafil mittels H⁺-Gradient. Die besten Einschlussraten wurden unter folgenden Bedingungen erzielt. Die Lipide (molares Verhältnis DPPC : Cholesterin = 55 : 45; in Summe 13 bis 15 µmol je ml wässrige Phase) wurden in Ethanol gelöst und diese Lösung in 125 mM Ammoniumsulfatlösung injiziert. Nach spontaner Vesikelbildung wurde die verbliebene, externe Ammoniumsulfatlösung durch eine 5% Glucoselösung ersetzt und Sildenafilcitrat zugegeben. Auf diese Weise bildete sich ein H⁺-Gradient zwischen Innen- und Aussenseite der Lipidvesikel aus. pH-Werte unter 2,5 sind aufgrund einsetzender Hydrolyseprobleme weniger geeignet, ebenso sind pH-Werte grösser 5,5 aufgrund des immer flacher werdenden H⁺-Gradienten nicht bevorzugt. Eine wässrige 125 mM Ammonisumsulfatlösung hat typischerweise einen pH-Wert im Bereich von ca. 5 - 5,5.

Nach Entfernung von nicht-eingeschlossenem Sildenafil mittels Gelfiltration, wurden sowohl die Wirkstoff- als auch die Lipidgehalte mittels rp-HPLC bestimmt. Liposomengrösse und -verteilung wurden mittels Photonenkorrelationsspektroskopie (PCS) bestimmt.

Abhängig von der Vesikelgrösse konnten mit diesem Verfahren der aktiven Beladung Einschlussraten (dargestellt als Sildenafil/Lipid-Verhältnis) von 160 bis 230 nmol Sildenafil pro µmol Gesamtlipide ( = DPPC + Cholesterin) erzielt werden. Umgerechnet ergibt dies Werte von 1000 - 1500 µg Wirkstoff pro ml liposomale Suspension.

Um eine Erhöhung der Menge an liposomal eingeschlossenem Sildenafil zu erreichen, wurde der Sildenafilgehalt in der Glucoselösung erhöht. Es zeigte sich jedoch, dass ein Überschuss an Wirkstoff das Verhältnis Wirkstoff/Lipid nicht verbessern konnte. Die effektive Beladungsmenge bei aktiver externer Beladung über einen H⁺-Gradienten scheint also in erster Linie vom Gradienten und weniger von der vorgelegten Wirkstoffkonzentration abhängig zu sein.

### b) einstufige Variante:

Die Lipide (DPPC : Cholesterin = 55 : 45 Mol%) wurden in Ethanol gelöst und die Lösung in eine Sildenafil/Ammoniumsulfatlösung (pH 3,5 - 4,5) injiziert, worauf unmittelbar nach spontan erfolgter Vesikelbildung eine 5% Glucoselösung (pH 7), die weiteres Sildenafilcitrat enthielt, zwecks Verdünnung und Alkalisierung der Reaktionsmischung, d.h. der entstandenen Liposomensuspension, zugesetzt wurde. Durch die Herstellung dieses H⁺-Gradienten unmittelbar nach der Vesikelbildung wird Sildenafil nicht nur in einem Schritt in die Liposomen aufgenommen, sondern dort auch stabil zurückgehalten. Die Menge an solcherart liposomal aufgenommenem Sildenafil lag - abhängig vom pH-Wert bzw. H⁺-Gradienten - ebenfalls in einem Bereich von ca. 160 bis 230 nmol Sildenafil pro µmol Gesamtlipide (DPPC + Cholesterin).

In analoger Art und Weise wurden auch die Wirkstoffe Tadalafil und Vardenafil in Liposomen geladen.

Zu Vergleichszwecken wurde unter ähnlichen Verfahrensbedingungen auch liposomal inkorporiertes Prostaglandin E1 hergestellt. Zu den Anwendungseffekten siehe Beispiel 2.

### Beispiel 2: Anwendung des liposomal inkorporierten Wirkstoffes

### A. Sildenafil, Tadalafil, Vardenafil in Liposomen

Als pharmazeutische Zusammensetzung für den Einsatz im Humanexperiment wurde eine Formulierung von 0,5 mg der jeweiligen Substanz (gerechnet als salzfreier Wirkstoff) in Liposomen pro 1 ml Hydrogel Carbopol 981 NF gewählt und von Probanden in einer Applikationsmenge von 0,5 - 1,5 ml pro Anwendung eingesetzt, wobei Sildenafil und Vardenafil jeweils in Form ihrer sauren Salze (Sildenafilcitrat bzw. Vardenafil-Hydrochloridtrihydrat) eingesetzt wurden.

Das Gel wurde von männlichen Probanden durch äusserliches Auftragen auf den Penis, von weiblichen Probanden durch äusserliche vaginale und/oder klitorale Aufbringung angewandt.

### Resultate:

### a) männlich:

Bereits kurz, d.h. innerhalb von wenigen Minuten, nach dem Auftragen des Präparates verspürten die Probanden ein angenehmes, warmes Gefühl, stärkere sexuelle Erregbarkeit. Es stellte sich in der Folge eine raschere und stärkere Erektion und deutlich länger anhaltende Steifigkeit des Penis im Vergleich zu den zuvor üblichen bzw. gewohnten Effekten ohne Anwendung des Präparates ein. Dabei wurden mit den drei Wirkstoffpräparaten ähnliche Wirkungen erzielt.

### b) weiblich:

Unmittelbar nach vaginaler/klitoraler Applikation des liposomalen Wirkstoff-Gels setzt die Wirkung ein, die eine erhöhte Durchblutung bewirkt, wodurch ein angenehmes, warmes Gefühl entsteht und in der Folge eine erhöhte Produktion an Vaginalsekret festgestellt wurde. Darüber hinaus berichteten die Probandinnen von leichten Kontraktionen der Vaginalmuskulatur (ähnlich wie beim Orgasmus), einem verstärkten sexuellen Empfinden beim Geschlechtsverkehr sowie einem stärkeren Empfinden des Orgasmus.

Durch die Anwendung des liposomalen Wirkstoff-Präparates werde nicht nur eine sexuelle Stimulation und eine Verstärkung des Lustempfindens bewirkt, sondern auch eine beschleunigte Reizauslösung und damit ein rascheres Erreichen des Orgasmus erzielt.

Wirkungsdauer nach einmaliger Applikation: Einsetzender Effekt sofort nach Aufbringen des Präpates, wobei die vorher beschriebenen Empfindungen bis zu 3,5 Stunden andauern. Alle drei Präparate zeigten eine deutliche Wirkung; ein wesentlicher, spürbarer Unterschied zwischen den drei verschiedenen Wirkstoffpräparaten wurde von den Probandinnen nicht berichtet.

Entgegen den Erkenntnissen aus den Pfizerstudien an Frauen (siehe New York Times, 28 Februar 2004, "Pfizer Gives Up Testing Viagra on Women") scheinen jedenfalls die erfindungsgemässen, liposomalen Wirkstoffpräparate bei äusserlicher Anwendung auch bei Frauen eindeutig wirksam zu sein. Sie können daher auch zur therapeutischen Behandlung von Sexualstörungen der Frau (female sexual dysfunction, FSD) wie beispielsweise zur Therapie der weiblichen Erregungsstörung (female sexual arousal disorder, FSAD), eingesetzt werden.

### B. Prostaglandin E1 in Liposomen

Ähnliche Wirkungen wurden von den weiblichen Probanden auch nach Applikation von liposomalem Prostaglandin E1 beschrieben. Die angewandte Dosierung war dabei mit 0,1 mg - 0,5mg je 1 ml Gel leicht unter der Dosierung von Sildenafil, Tadalafil und Vardenafil.

## Patentansprüche

1. Pharmazeutische Zusammensetzung zur topischen Applikation, mit einem in Liposomen eingeschlossenen Wirkstoff, der direkt oder indirekt eine relaxierende Wirkung auf die glatte Muskulatur ausübt, **dadurch gekennzeichnet, dass** die Liposomen einen molaren Anteil von Cholesterin von 0 - 50 %, vorzugsweise von 30 - 45 %, bezogen auf die Gesamtlipidmenge, besitzen sowie in ihrem Inneren ein wässriges Milieu mit einem pH-Wert im Bereich von 2,5 - 5,5 aufweisen und darin mindestens einen protonierten Wirkstoff aus der Gruppe der Prostaglandine, Adenylatcyclasen, cAMP, AMP, ATP, NO-Synthetasen, Stickstoffmonoxid (NO), NO-Verbindungen, Nitrate, Guanylat-, cyclasen, cGMP, GMP, GTP und Phosphodiesterasen in einer Konzentration von mindestens 100 nmol je µmol Lipid enthalten, und wobei ausserhalb der Liposomen ein wässriges Milieu mit neutralem oder alkalischem pH-Wert vorliegt.

2. Zusammensetzung nach Anspruch 1, **dadurch gekennzeichnet, dass** innerhalb der Liposomen ein wässriges Milieu mit einem pH-Wert im Bereich von 5,0 -5,5 und ausserhalb der Liposomen ein wässriges Milieu mit einem pH-Wert von 7 bis 8 vorliegt.

3. Zusammensetzung nach Anspruch 1 oder 2, **dadurch gekennzeichnet, dass** der Wirkstoff aus der Gruppe Sildenafil, Tadalafil und Vardenafil, sowie deren saurer Salze, ausgewählt ist.

4. Zusammensetzung nach Anspruch 3, **dadurch gekennzeichnet, dass** der Wirkstoff Sildenafilcitrat und das wässrige Milieu innerhalb der Liposomen ein Ammoniumsulfat-Puffersystem ist.

5. Zusammensetzung nach einem der Ansprüche 1 bis 4, **dadurch gekennzeichnet, dass** das wässrige Milieu innerhalb der Liposomen ein Ammoniumsulfat-Puffersystem und das Milieu ausserhalb der Liposomen eine 5% Glucoselösung ist.

6. Zusammensetzung nach einem der Ansprüche 1 bis 3, **dadurch gekennzeichnet, dass** das wässrige Milieu innerhalb der Liposomen ein Citrat-Puffersystem und das Milieu ausserhalb der Liposomen ein Zitronensäure/Natriumcarbonat-Puffer ist.

7. Zusammensetzung nach einem der Ansprüche 1 bis 6, **dadurch gekennzeichnet, dass** die Liposomen Phospholipide mit einer Acylkettenlänge von mindestens 14, vorzugsweise mindestens 16, Kohlenstoffatomen enthalten.

8. Zusammensetzung nach einem der Ansprüche 1 bis 7, **dadurch gekennzeichnet, dass** die Liposomen eine mittlere Grösse im Bereich von 150 bis 500 nm aufweisen und den Wirkstoff in einer Konzentration von 150 - 400 nmol je µmol Lipid enthalten.

9. Zusammensetzung nach einem der Ansprüche 1 bis 8, **dadurch gekennzeichnet, dass** sie als Suspension, Lotion, Emulsion, Tinktur, Spray, Gel, Creme oder Salbe, vorzugsweise in aseptischer Form, vorliegt.

10. Verfahren zur Herstellung einer in den Ansprüchen 1 bis 19 definierten pharmazeutischen Zusammensetzung, **dadurch gekennzeichnet, dass** durch Injektion einer ethanolischen Lipidphase mit einem molaren Anteil von Cholesterin von 0 - 50 %, vorzugsweise von 30 - 45 %%, bezogen auf die Gesamtlipidmenge, in eine wässrige Phase mit einem pH-Wert im Bereich von 2,5 - 5,5 spontan Liposomen mit einem wässrigen Milieu in ihrem Inneren erzeugt werden, worauf die wässrige Phase modifiziert, nämlich verdünnt, ausgetauscht, neutralisiert oder alkalisiert wird, sodass sich zwischen der Innen- und Aussenseite der Liposomen ein H⁺-Gradient ausbildet und wobei ein protonierbarer Wirkstoff aus der Gruppe der Prostaglandine, Adenylatcyclasen, cAMP, AMP, ATP, NO-Synthetasen, Stickstoffmonoxid (NO), NO-Verbindungen, Nitrate, Guanylatcyclasen, cGMP, GMP, GTP und Phosphodiesterasen
a) in der wässrigen Phase vorgelegt und im Zuge der spontan erfolgenden Liposomenbildung in die Liposomen aufgenommen wird, und/oder
b) erst nach erfolgter Vesikelbildung der modifizierten wässrigen Phase zugesetzt wird und entlang dem H⁺-Gradienten in die Liposomen migriert,
wobei Liposomen gebildet werden, die den Wirkstoff in einer Konzentration von mindestens 100, insbesondere von 150 - 400 nmol je µmol Lipid enthalten.

11. Verfahren nach Anspruch 10, **dadurch gekennzeichnet, dass** die Modifikation der wässrigen Phase unmittelbar nach erfolgter Liposomenbildung durch Verdünnen mit einem neutralen oder alkalischen Puffer vorgenommen wird, wodurch ein pH-Wert von 7 - 8 erreicht wird.

12. Verfahren nach Anspruch 11, **dadurch gekennzeichnet, dass** die wässrige Phase vor der Modifikation einen pH-Wert von 3,5 - 4,5 aufweist und Ammoniumsulfat enthält und die Modifikation durch Verdünnen der wässrigen Phase mit einer 5% Glucoselösung vorgenommen wird.

13. Verfahren nach einem der Ansprüche 10 bis 12, **dadurch gekennzeichnet, dass** die pharmazeutische Zusammensetzung als Suspension, Lotion, Emulsion, Tinktur, Spray, Gel, Creme oder Salbe, vorzugsweise in aseptischer Form, hergestellt wird.

14. Pharmazeutische Zusammensetzung nach einem der Ansprüche 1 bis 9, zur Verwendung als Medikament für äusserliche, insbesondere topische transdermale und/oder transmucosale, Anwendungen im Genitalbereich.

15. Pharmazeutische Zusammensetzung nach Anspruch 14, zur Verwendung in der Prophylaxe und/oder Therapie erektiler Störungen beim Mann, in der Behandlung sexueller Störungen der Frau, insbesondere in der Therapie von weiblichen Erregungsstörungen (FSAD, female sexual arousal disorder), oder zur Steigerung des sexuellen Lustempfindens.

## Claims

1. Pharmaceutical composition for topical application, comprising an active ingredient which is enclosed in liposomes and has a direct or indirect relaxing effect on smooth muscles, **characterized in that** the liposomes have a molar fraction of cholesterol of 0 - 50 %, preferably of 30 - 45 %, based on the total amount of lipids, and have, in their inferior, an aqueous medium having a pH in the range of 2.5 - 5.5 and contain therein at least one protonated active ingredient from the group consisting of the prostaglandins, adenylate cyclases, cAMP, AMP, ATP, NO synthetases, nitric oxide (NO), NO compounds, nitrates, guanylate cyclases, cGMP, GMP, GTP and phosphodiesterases in a concentration of at least 100 nmol per µmol of lipid, an aqueous medium having a neutral or alkaline pH being present outside the liposomes.

2. Composition according to Claim 1, **characterized in that** an aqueous medium having a pH in the range of 5.0 - 5.5 is present inside the liposomes and an aqueous medium having a pH of 7 to 8 is present outside the liposomes.

3. Composition according to Claim 1 or 2, **characterized in that** the active ingredient is selected from the group consisting of sildenafil, tadalafil and vardenafil and the acidic salts thereof.

4. Composition according to Claim 3, **characterized in that** the active ingredient is sildenafil citrate and the aqueous medium within the liposomes is an ammonium sulphate buffer system.

5. Composition according to any of Claims 1 to 4, **characterized in that** the aqueous medium within the liposomes is an ammonium sulphate buffer system and the medium outside the liposomes is a 5 % glucose solution.

6. Composition according to any of Claims 1 to 3, **characterized in that** the aqueous medium within the liposomes is a citrate buffer system and the medium outside the liposomes is a citric acid/sodium carbonate buffer.

7. Composition according to any of Claims 1 to 6, **characterized in that** the liposomes contain phospholipids having an acyl chain length of at least 14, preferably at least 16, carbon atoms.

8. Composition according to any of Claims 1 to 7, **characterized in that** the liposomes have an average size in the range of 150 to 500 nm and contain the active ingredient in a concentration of 150 - 400 nmol per µmol of lipid.

9. Composition according to any of Claims 1 to 8, **characterized in that** it is present as a suspension, lotion, emulsion, tincture, spray, gel, cream or ointment, preferably in aseptic form.

10. Process for the preparation of a pharmaceutical composition defined in Claims 1 to 9, **characterized in that** liposomes having an aqueous medium in their interior are produced spontaneously by injection of an ethanolic lipid phase having a molar fraction of cholesterol of 0 - 50 %, preferably of 30 - 45 %, based on the total amount of lipids, into an aqueous phase having a pH in the range of 2.5 - 5.5, whereupon the aqueous phase is modified, namely diluted, exchanged, neutralized or rendered alkaline, so that a H⁺ gradient forms between the inside and outside of the liposomes, a protonatable active ingredient from the group consisting of the prostaglandins, adenylate cyclases, cAMP, AMP, ADP, NO synthetases, nitric oxide (NO), NO compounds, nitrates, guanylate cyclases, cGMP, GMP, GTP and phosphodiesterases
a) being initially introduced in the aqueous phase and being absorbed into the Liposomes in the course of the spontaneous liposome formation, and/or
b) being added only after vesicle formation to the modified aqueous phase and migrating along the H⁺ gradient into the liposomes,
whereby liposomes are formed which contain the active ingredient in a concentration of at least 100, in particular of 150 - 400, nmol per µmol of lipid.

11. Process according to Claim 10, **characterized in that** the modification of the aqueous phase is carried out immediately after liposome formation by dilution with a neutral or alkaline buffer, with the result that a pH of 7 - 8 is reached.

12. Process according to Claim 11, **characterized in that** the aqueous phase has a pH of 3.5 - 4.5 before the modification and contains ammonium sulphate and the modification is carried out by dilution of the aqueous phase with a 5 % glucose solution.

13. Process according to any of Claims 10 to 12, **characterized in that** the pharmaceutical composition is prepared as a suspension, lotion, emulsion, tincture, spray, gel, cream or ointment, preferably in aseptic form.

14. Pharmaceutical composition according to any of Claims 1 to 9 for use as a medicament for external, in particular topical transdermal and/or transmucosal, applications in the genital area.

15. Pharmaceutical composition according to Claim 14 for use in the prophylaxis and/or therapy of male erectile dysfunction, in the treatment of female sexual dysfunction, in particular in the therapy of female sexual arousal disorder (FSAD) or for increasing the sensation of sexual pleasure.

## Revendications

1. Composition pharmaceutique pour application topique, avec une principe actif, inclus dans des liposomes, exerçant, directement ou indirectement, un effet relaxant sur la musculature lisse, **caractérisée en ce que** les liposomes comprennent une proportion molaire de cholestérol dans la fourchette de 0 à 50 %, de préférence de 30 à 45 %, par rapport à la quantité globale de lipides, ainsi que, intérieurement, présentent un milieu aqueux ayant une valeur de pH dans la fourchette de 2,5 à 5,5 et, dans celui-ci, au moins un principe actif protoné, issu du groupe de la prostaglandine, des adénylatcyclases, cAMP, AMP, ATP, NO-synthétases, monoxyde d'azote (NO), combinaisons NO, nitrates, guanylate-cyclases, cGMP, GMP, GTP et phosphodiestérases, en une concentration d'au moins 100 nmoles par µmole de lipide, et où un milieu aqueux à valeur de pH neutre ou alcaline est présent à l'extérieur des liposomes.

2. Composition selon la revendication 1, **caractérisée en ce que**, à l'intérieur des liposomes, est présent un milieu aqueux à valeur de pH dans la fourchette de 5,0 à 5,5 et, à l'extérieur des liposomes, est présent un milieu aqueux à valeur de pH dans la fourchette de 7 à 8.

3. Composition selon la revendication 1 ou 2, **caractérisée en ce que** le principe actif est sélectionné dans le groupe des sildénafil, tadalafil et vardénafil, ainsi que leurs sels acides.

4. Composition selon la revendication 3, **caractérisée en ce que** le principe actif est du sildénafil citrate et le milieu aqueux à l'intérieur des liposomes est un système tampon au sulfate d'ammonium.

5. Composition selon l'une des revendications 1 à 4, **caractérisée en ce que** le milieu aqueux à l'intérieur des liposomes est un système tampon au sulfate d'ammonium et le milieu à l'extérieur des liposomes est une solution de glucose à 5 %.

6. Composition selon l'une des revendications 1 à 3, **caractérisée en ce que** le milieu aqueux à l'intérieur des liposomes est un système tampon au citrate et le milieu à l'extérieur des liposomes est une tampon acide citrique/carbonate de sodium.

7. Composition selon l'une des revendications 1 à 6, **caractérisée en ce que** les liposomes contiennent des phospholipides ayant une longueur de la chaîne acyl d'au moins 14, de préférence d'au moins 16, atomes de carbone.

8. Composition selon l'une des revendications 1 à 7, **caractérisée en ce que** les liposomes présentent une taille moyenne dans la fourchette de 150 à 500 nm et contiennent le principe actif en une concentration dans la fourchette de 150 à 400 nmoles par µmole de lipide.

9. Composition selon l'une des revendications 1 à 8, **caractérisée en ce qu'**elle se présente sous forme de suspension, lotion, émulsion, teinture, spray, gel, crème ou onguent, de préférence sous forme aseptique.

10. Procédé de préparation d'une composition pharmaceutique définie aux revendications 1 à 19, **caractérisé en ce que** des liposomes spontanés, avec un milieu aqueux à l'intérieur de ceux-ci, sont produits par injection d'une phase lipide éthanolique ayant une proportion molaire de cholestérol de 0 à 50 %, de préférence de 30 à 45 %, rapportée à la quantité globale de lipide, dans une phase aqueuse ayant une valeur de pH dans la fourchette de 2,5 à 5,5, suite à quoi la phase aqueuse est modifiée, précisément diluée, soumise à échange, neutralisée ou alkalisée, de manière qu'un gradient H⁺ se constitue entre les côtés intérieur et extérieur des liposomes, et où un principe actif protonable, issu du groupe de la prostaglandine, des adénylatcyclases, cAMP, AMP, ATP, NO-synthétases, monoxyde d'azote (NO), combinaisons NO, nitrates, guanylate-cyclases, cGMP, GMP, GTP et phosphodiestérases,
a) se présente dans la phase aqueuse et est absorbé au cours de la formation, s'effectuant spontanément, des liposomes, et/ou
b) ensuite, une fois effectuée la formation de vésicules, est ajouté à la phase aqueuse modifiée et migre, le long du gradient H+, dans les liposomes,
dont le resultat et la formation de liposomes qui contiennent le principe actif en une concentration d'au moins 100, en particulier dans la fourchette de 150 à 400 nmoles par µmole de lipide.

11. Procédé selon la revendication 10, **caractérisé en ce que** la modification de la phase aqueuse est effectuée directement après avoir effectue la formation des liposomes, par dilution avec un tampon neutre ou alcalin, faisant qu'une valeur de pH de 7 à 8 est atteinte.

12. Procédé selon la, revendication 11, **caractérisé en ce que**, avant la modification, la phase aqueuse présente une valeur de pH de 3,5 à 4,5 et contient du sulfate d'ammonium, et la modification est effectuée par dilution de la phase aqueuse avec une solution de glucose à 5 %.

13. Procédé selon l'une des revendications 10 à 12, **caractérisé en ce que** la composition pharmaceutique est préparée sous forme de suspension, lotion, émulsion, teinture, spray, gel, crème ou onguent, de préférence sous forme aseptique.

14. Composition pharmaceutique selon l'une des revendications 1 à 9, pour utilisation en tant que médicament, pour applications externes, en particulier topiques, transdermales et/ou par voie transmuqueuse, dans la zone génitale.

15. Composition pharmaceutique selon la revendication 14, pour utilisation dans la prophylaxie et/ou la thérapie des troubles érectiles chez l'homme, dans le traitement des troubles sexuels de la femme, en particulier dans la thérapie des désordres de l'excitation féminine (FSAD, female sexual arousal disorder - désordre sexuel femelle d'éveil), ou pour l'augmentation de la sensation de plaisir sexuelle.
